# EUROPEAN PATENT APPLICATION

(11) **EP 1 378 201 A1**
(43) Date of publication of application: **07.01.2004**
(21) Application number: 03006668.2
(22) Date of filing: 25.03.2003
(51) Int. Cl.: A61B 6/14

(54) **Dental radiographic image acquisition and display unit**

(30) Priority: 05.07.2002 IT BO20020440
(71) Applicant: CEFLA SOC. COOP. A R.L., I-40026 Imola, Bologna (IT)
(72) Inventor: Nanni, Eros, 40023 Castel Guelfo di Bologna (IT); Pradella, Riccardo, 40026 Imola (IT)
(74) Representative: Cerbaro, Elena, Dr.

(57) **Abstract**

In a dental radiographic image acquisition and display unit, a radiographic sensor (5) acquires a dental radiographic image generated by an X-ray emitting device (2), and transfers the image to a computer (6) by means of a radio signal transmitting device (8), which employs an interface (9) housed in the computer (6) and operating to a standard commercial protocol.

## Description

The present invention relates to a dental radiographic image acquisition and display unit.

In dentistry, a dental radiographic image acquisition and display unit is employed comprising an X-ray emitting device; a radiographic sensor for acquiring a dental radiographic image; and a computer for storing and displaying the image.

The sensor and computer normally communicate via respective communication devices connected to one another over a relatively long electric cable, which impedes the freedom of movement of the user as they work.

Another drawback of known sensors lies in compulsory use of the proprietary image acquisition software supplied with the sensor, as opposed to selective use of other types of image acquisition software possibly already in the user's possession.

It is an object of the present invention to provide a dental radiographic image acquisition and display unit designed to eliminate the aforementioned drawbacks.

According to the present invention, there is provided a dental radiographic image acquisition and display unit as claimed in Claim 1.

A non-limiting embodiment of the present invention will be described by way of example with reference to the accompanying drawings, in which:
Figure 1 shows an exploded view in perspective of a preferred embodiment of the unit according to the present invention;
Figure 2 shows an operating diagram of the Figure 1 unit.

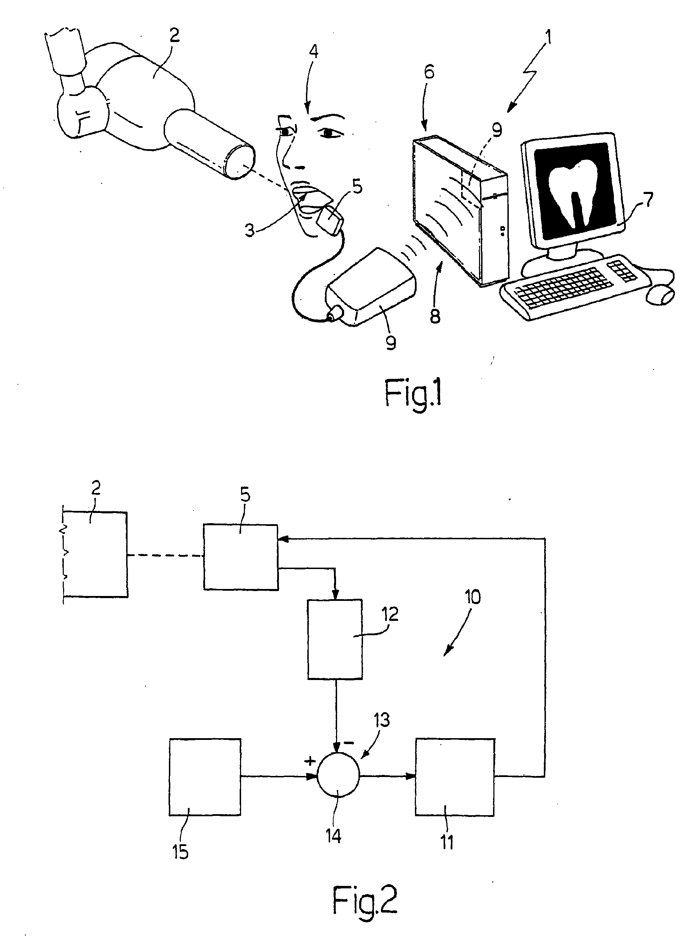

Number 1 in Figure 1 indicates as a whole a dental radiographic image acquisition and display unit comprising an emitting device 2 for directing an X-ray beam onto the oral cavity 3 of a patient 4; a radiographic sensor 5 for acquiring a radiographic image of oral cavity 3 generated by device 2; a computer 6 for storing and displaying on a monitor 7 the radiographic image acquired by sensor 5; and a transmission device 8 for connecting sensor 5 and computer 6.

Device 8 comprises two electronic interfaces 9 - one associated with sensor 5 and powered by at least one battery (not shown), and the other associated with computer 6 - which communicate with each other by radio signals to transfer the radiographic image acquired by sensor 5 to computer 6.

Each interface 9 is preferably one employing a commercial digital standard operating in a 300 MHz to 10 GHz frequency range.

The electronic interface 9 connected to computer 6 operates to a standard commercial image management protocol (e.g. "TWAIN" or "STI" - short for "Still Image application programming interface"). Using a standard commercial communication protocol to connect sensor 5 to standard image acquisition software on computer 6 allows the user to employ sensor 5 without necessarily using the proprietary software supplied with it. Sensor 5 can thus be connected easily to any standard image acquisition software (even not specifically designed for dentistry), so that any past data acquired using existing user software can be preserved, and need not be reloaded using the proprietary software supplied with sensor 5.

Sensor 5 comprises a CCD or CMOS sensor, and a scintillating device (not shown) connected to the CCD or CMOS sensor.

With reference to Figure 2, unit 1 also comprises a control unit 10, in turn comprising a drive device 11 for supplying he necessary signals to radiographic sensor 5, and a measuring device 12 for real-time analyzing the output signal from sensor 5 to determine the exposure values of various sample areas of sensor 5, and the maximum exposure value of the areas sampled.

Unit 10 also comprises a control circuit 13 based on a known microprocessor 14, which compares the maximum exposure value of the sample areas of sensor 5 (the maximum value supplied by measuring device 12) with a typical saturation value expected of a dental X-ray and stored in a storage device 15. Depending on the outcome of the comparison, control circuit 13 supplies drive device 11 with a signal to interrupt acquisition by sensor 5 when the maximum exposure value of the sample areas of sensor 5 substantially nears saturation, thus obtaining a good-quality image, even in the event of excessive exposure times being set by the user.

Once acquisition by sensor 5 is completed, the whole surface of sensor 5 is scanned, and the individual pixel values detected are transferred to a memory (not shown) where they are stored temporarily pending any digital processing and transmission to computer 6 by device 8.

## Claims

1. A dental radiographic image acquisition and display unit, comprising an X-ray emitting device (2); a radiographic sensor (5) for acquiring a dental radiographic image; a computer (6) for storing and displaying said image; and transmission means (8) for transmitting the image from the sensor (5) to the computer (6); **characterized in that** the transmission means (8) are radio signal transmission means.

2. A unit as claimed in Claim 1, wherein said transmission means (8) comprise two communication devices (9) associated with the sensor (5) and the computer (6) respectively.

3. A unit as claimed in Claim 2, wherein each said communication device (9) comprises an interface employing a commercial digital standard operating in a 300 MHz to 10 GHz frequency range.

4. A unit as claimed in Claim 2 or 3, wherein the communication device (9) associated with the computer (6) operates to a standard commercial image management protocol.

5. A unit as claimed in Claim 4, wherein said standard commercial protocol is the TWAIN protocol.

6. A unit as claimed in Claim 4, wherein said standard commercial protocol is the STI protocol.

7. A unit as claimed in any one of the foregoing Claims, wherein said sensor (5) comprises a CCD sensor, and a scintillating device connected to the CCD sensor.

8. A unit as claimed in any one of Claims 1 to 6, wherein said sensor (5) comprises a CMOS sensor, and a scintillating device connected to the CMOS sensor.

9. A unit as claimed in any one of the foregoing Claims, and also comprising control means (10) for selectively controlling an exposure time of said sensor (5); the control means (10) comprising a measuring device (12) connected to the sensor (5) and for real-time measuring a maximum exposure value of the image during acquisition of the image by the sensor (5), and a drive device (11) for interrupting acquisition of the image by the sensor (5) when the measured maximum exposure value reaches a predetermined saturation value.

10. A dental radiographic image acquisition and display unit, comprising an X-ray emitting device (2); a radiographic sensor (5) for acquiring a dental radiographic image; a computer (6) for storing and displaying said image; and transmission means (8) for transmitting the image from the sensor (5) to the computer (6); **characterized in that** the transmission means (8) are radio signal transmission means comprising a communication device (9) associated with the computer (6) and operating to a standard commercial protocol.
